(19) Europäisches Patentamt
European Patent Office
Office européen des brevets

(11) EP 1 704 896 B1

(12) **FASCICULE DE BREVET EUROPEEN**

(45) Date de publication et mention de la délivrance du brevet:
**01.12.2010 Bulletin 2010/48**

(51) Int Cl.:
***A61Q 1/02*** *(2006.01)* ***A61Q 1/04*** *(2006.01)*
***A61Q 1/06*** *(2006.01)* ***A61Q 1/10*** *(2006.01)*
***A61K 8/73*** *(2006.01)* ***A61K 8/81*** *(2006.01)*

(21) Numéro de dépôt: **06300249.7**

(22) Date de dépôt: **20.03.2006**

(54) **Kit de maquillage et/ou de soin susceptible de procurer un effet volumateur**

Kit zum Schminken und/oder zur Pflege zur Bereitstellung eines Volumeneffekts

Kit for make-up and/or for care capable of providing a voluminous effect

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **24.03.2005 FR 0550774**

(43) Date de publication de la demande:
**27.09.2006 Bulletin 2006/39**

(73) Titulaire: **L'Oréal**
**75008 Paris (FR)**

(72) Inventeurs:
- **Pays, Karl**
  **94410, Saint-Maurice (FR)**
- **Bichon, Yohann**
  **94700 Maison-Alfort (FR)**

(74) Mandataire: **Le Coupanec, Pascale A.M.P. et al**
**Nony & Associés**
**3, rue de Penthièvre**
**75008 Paris (FR)**

(56) Documents cités:
**EP-A- 1 069 151**
**EP-A- 1 249 223**
**EP-A- 1 396 257**
**EP-A- 1 415 640**
**EP-A- 1 518 534**
**WO-A-99/52499**
**WO-A-2004/103302**
**FR-A- 2 785 801**
**FR-A- 2 859 101**

Remarques:
Le dossier contient des informations techniques présentées postérieurement au dépôt de la demande et ne figurant pas dans le présent fascicule.

EP 1 704 896 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne un kit de maquillage notamment de la peau, des lèvres et/ou des fibres kératiniques comprenant au moins une composition aqueuse et au moins une composition cosmétique à phase continue huileuse, susceptible de procurer un effet volumateur lors de la mise en contact desdites compositions.

Plus particulièrement, le kit de maquillage selon l'invention, peut constituer un produit de maquillage du visage, du corps et/ou des lèvres.

La présente invention se rapporte en outre au maquillage des fibres kératiniques, comme les cils, les sourcils et les cheveux, et plus particulièrement au maquillage des cils.

Le kit de maquillage selon l'invention peut se présenter sous la forme d'un kit de maquillage pour les cils, pour les sourcils, ou d'un kit de maquillage des cheveux. Plus spécialement, l'invention porte sur un mascara. Il peut notamment s'agir d'un kit de maquillage ou bien d'un kit de traitement des cils.

D'une manière générale, les compositions de maquillage de fibres kératiniques et notamment de cils comprennent une phase grasse liquide (huiles) contenant un structurant huileux qui peut être une cire, un polymère, en particulier un polymère semi-cristallin ou un gélifiant lipophile.

Il existe en pratique essentiellement deux types de formulation de mascara, à savoir d'une part, des mascaras à phase continue aqueuse, dits "mascaras émulsion", se présentant sous forme d'émulsion de cires dans l'eau et, d'autre part, des mascaras à phase continue solvant ou huile, anhydres ou à faible teneur en eau et/ou solvants hydrosolubles, dits "mascaras waterproof", formulés à l'état de dispersion de cires dans des solvants non aqueux.

Par composition à phase continue aqueuse, on entend que la composition présente une conductivité, mesurée à 25 °C, supérieure ou égale à 23 $\mu$S/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

Par composition à phase continue solvant, on entend que la composition présente une conductivité, mesurée à 25 °C, inférieure à 23 $\mu$S/cm (microSiemens/cm), la conductivité étant mesurée comme indiquée ci-dessus.

"Conférer du volume", encore ci-après qualifié d'effet volumateur, est un effet fréquemment recherché dans le domaine cosmétique.

Dans le domaine du maquillage des cils, il a par exemple été proposé des formulations de mascara très concentrées, à extrait sec élevé, permettant de déposer une forte épaisseur de matière. Le principe de ces mascaras épaississants est en d'autres termes de déposer le maximum de matière sur les cils. Cependant, de telles formulations présentent l'inconvénient d'être très consistantes et donc difficilement applicables. L'inconvénient majeur est la formation de "paquets" jugés inesthétiques. Toujours dans le domaine des mascaras il a, à l'inverse, été proposé des formulations très fluides qui par superposition de couches permettent également l'obtention d'un effet volumateur. Toutefois là encore, les conditions d'obtention de l'effet volumateur ne sont pas totalement satisfaisantes car leur mise en oeuvre est délicate et longue.

Pour les lèvres, l'effet volumateur a jusqu'à présent principalement été obtenu soit en dessinant un contour des lèvres plus large que le contour naturel, soit en jouant sur des effets optiques par exemple à l'aide de compositions brillantes.

Une autre alternative a consisté à faire usage, dans des compositions cosmétiques, de polymères dotés d'une forte capacité d'absorption de l'eau. La mise en contact de la composition, appliquée sur son site de maquillage, avec de l'eau, provoque une augmentation de son volume initial, générant ainsi l'effet volumateur recherché. De telles compositions sont notamment décrites dans les documents US 6,045,783 et EP 1 228 747. Les compositions à effet volumateur utilisant ces polymères sont cependant peu satisfaisantes car l'obtention d'un gonflement significatif nécessite l'apport d'une quantité d'eau assez importante.

Les documents FR 2 785 801, EP 1 152 022, FR 2 774 996, WO 95/35089 et WO 99/36445 décrivent des compositions épaississantes, dites "latex épaississant" ou "agent épaississant" ou encore "latex inverse".

Les demandes de brevet FR 2 785 801 et FR 2 774 996 divulguent notamment des compositions comprenant une phase aqueuse, une phase huileuse, un agent émulsifiant de type H/E (huile dans eau) et un agent émulsifiant de type E/H (eau dans huile) ainsi qu'un polyélectrolyte anionique, branché ou réticulé, à base d'un monomère possédant une fonction acide fort.

Le document WO 99/52499 décrit principalement des rouges à lèvres comprenant un polyacrylate de sodium dans le but de produire un effet volumateur.

On a maintenant découvert qu'il était possible d'obtenir des kits de maquillage capables de générer un effet volumateur particulièrement avantageux tout en maintenant une bonne séparation des cils.

Selon un premier aspect, la présente invention concerne un kit de maquillage et/ou de soin de la peau, des lèvres et/ou des fibres kératiniques comprenant au moins :

- une composition cosmétique à phase continue huileuse comprenant au moins un polyélectrolyte et au moins un agent tensioactif de HLB supérieur ou égal à 6 à 25°C, et
- une composition aqueuse.

Les inventeurs ont ainsi observé que la mise en contact, notamment au cous du maquillage, d'une composition aqueuse avec une composition cosmétique conforme à la présente invention, quel que soit l'ordre dans lequel ces deux compositions sont déposées, permet de générer un effet volumateur particulièrement avantageux en terme de maquillage.

Selon un deuxième aspect, la présente invention concerne un procédé cosmétique de maquillage et/ou de soin non thérapeutique de la peau, des lèvres et/ou des fibres kératiniques comprenant au moins une étape d'application sur la peau, les lèvres et/ou les fibres kératiniques d'une composition cosmétique telle que définie précédemment et au moins une étape d'application d'une composition aqueuse.

Selon un troisième aspect, la présente invention concerne l'utilisation d'au moins un polyélectrolyte et au moins un agent tensioactif de HLB supérieur ou égal à 6 à 25°C pour la préparation d'un kit de maquillage de la peau, des lèvres et/ou des fibres kératiniques comprenant au moins une composition cosmétique à phase continue huileuse et au moins une composition aqueuse, à titre d'additifs pour ladite composition cosmétique à phase continue huileuse pour obtenir un effet volumateur lorsque lesdites compositions sont mises en contact, notamment au cours du maquillage.

Comme il ressort des exemples ci-après, les kits de maquillage selon l'invention manifestent de tels avantages techniques.

Dans le cadre de la présente invention, par "fibres kératiniques" on entend notamment les cheveux, les cils et les sourcils. De plus, le maquillage de la peau s'entend notamment du maquillage du corps, des mains, du cou ou du visage.

Les kits de maquillage conformes à l'invention comprennent un milieu physiologiquement acceptable, notamment cosmétiquement acceptable, c'est-à-dire un milieu compatible en particulier avec les fibres kératiniques telles que les cheveux, les cils, les sourcils.

Par "cosmétiquement acceptable", on entend, dans le cadre de la présente invention, un composé dont l'utilisation est compatible avec une application sur les matières kératiniques.

Dans un soucis de simplification, sauf indication contraire, le terme "composition cosmétique à phase continue huileuse" ou "composition cosmétique" ou "composition cosmétique conforme à l'invention" ou encore "composition cosmétique comprise dans le kit" désigne la composition cosmétique à phase continue huileuse comprenant au moins un polyélectrolyte et au moins un agent tensioactif de HLB supérieur ou égal à 6 à 25°C, telle que comprise dans le kit de maquillage selon la présente invention.

Ainsi, l'utilisatrice peut obtenir l'effet volumateur recherché par au moins deux gestes d'application.

Dans le cadre de la présente invention, les termes "effet volumateur", "effet de gonflement" et "effet épaississant", qui qualifient le maquillage résultant de l'application des deux compositions comprise dans le kit revendiqué, sont employés indifféremment. On note que le terme "effet de gonflement" est particulièrement approprié au maquillage et/ou au soin des fibres kératiniques.

L'effet volumateur peut avantageusement se manifester par un effet de gonflement des cils. Cet effet de gonflement est d'autant plus manifeste lorsque la composition cosmétique conforme à l'invention est déposée en premier lieu, et la composition aqueuse est déposée en second lieu et lorsque la composition cosmétique conforme à l'invention contient des matières colorantes et la composition aqueuse est avantageusement transparente ou translucide. On peut alors, dans ce cas du maquillage des cils, observer à l'oeil nu un gonflement du gainage des cils dès lors que le dépôt de la composition aqueuse a eu lieu. Autrement dit la section du cil gainé, après application de la composition aqueuse, augmente comparativement à la section du cil gainé par la seule application de la composition cosmétique conforme à la présente invention.

L'effet épaississant se manifeste ainsi en particulier lorsque les deux compositions sont mises en contact, et de préférence sont soumises à un cisaillement, par exemple au cours du maquillage et notamment lors de l'application sur la peau, les lèvres et/ou les fibres kératiniques.

L'application peut notamment avoir lieu avec les doigts, ou à l'aide de tout moyen d'application, ou applicateur, tel que cela est décrit ci-dessous.

Ce mode de réalisation de l'invention ne présente toutefois aucun caractère limitatif, de sorte que l'on reste dans le cadre de la présente invention, notamment lorsque l'ordre d'application des compositions est différent.

Suivant l'ordre d'application, l'une ou l'autre des composition cosmétique ou composition aqueuse peut être qualifiée de "basecoat" ou de "topcoat".

**<u>POLYELECTROLYTE</u>**

Par "polyélectrolyte", on entend une substance macromoléculaire, qui a la faculté de se dissocier, lorsqu'elle

est dissoute dans l'eau ou dans tout autre milieu ionisant, pour donner au moins un ion. Autrement dit, un polyélectrolyte est un polymère comportant au moins un monomère ionisable.

En particulier, le polyélectrolyte peut donner des polyions, par exemple des polyanions, lorsqu'il est dissocié dans l'eau. Un polyélectrolyte peut être un polyacide, une polybase, un polysel ou polyampholyte. Dans le cadre de l'invention, il est de préférence un polyacide et de préférence un polyacide fort.

De façon préférée, le polyélectrolyte compris dans les compositions cosmétiques selon la présente invention est un polymère anionique branché et/ou réticulé.

De préférence, le polyélectrolyte est en outre apte à former en solution aqueuse, au-delà d'une concentration supérieure ou égale à 0,1 % en poids de matière sèche, de préférence ≥ 0,3 % en poids par rapport au poids total de la composition, un gel. Ce gel peut être caractérisé par rhéologie oscillatoire (v = 1 Hz) par un seuil d'écoulement $\tau_c$ au moins égal à 10 Pa.

De plus, lorsque la composition cosmétique selon la présente invention comprend un polymère filmogène, le polyélectrolyte se distingue avantageusement de ce polymère filmogène.

Les contre-ions des polyions formés lors de la dissociation peuvent être de toute nature, inorganique ou organique.

En particulier, lorsque le polyélectrolyte est un polymère anionique branché ou réticulé, les cations peuvent être des cations alcalins ou alcalino-terreux tels que le sodium ou le potassium ou encore l'ion ammonium.

Le cation sodium $Na^+$ est préféré, c'est pourquoi il est principalement cité dans la liste de polyélectrolytes qui va suivre, sans que cela constitue une quelconque limitation à ce contre-ion spécifique.

A titre de polyélectrolyte, on peut citer:

- le copolymère acrylamide / AMPS de Na tel que le Simulgel 600® sous forme d'émulsion contenant du polysorbate 80 à titre de tensioactif et contenant de l'isohexadécane à titre de phase huile, vendu par la société SEPPIC, ou encore le Simulgel EG® , le Simulgel A® et le Simulgel 501® vendus par la même société.

Le Simulgel 600® est notamment décrit dans le document FR 2 785 801. Il s'agit en réalité d'un latex inverse. Le polyélectrolyte AMPS est de l'acide 2-méthyl 2-[(1-oxo 2-propényl)amino] 1-propanesulfonique partiellement ou totalement salifié notamment sous forme de sel de sodium ou de sel d'ammonium compris à hauteur de 30 à 50 % en proportions molaires dans le mélange comprenant de l'AMPS ainsi qu'un acrylamide, contenu quant à lui à hauteur de 50 à 70 %.

- le glycolate sodique d'amidon réticulé sous forme de poudre,
- les polyacrylates de sodium tel que le Norsocryl S35® vendu par la société ATOFINA, ou le Cosmedia SP® vendu par la société COGNIS,
- les dérivés ionisables de polysaccharides tels que les sels de cellulose et les alginates de sodium,
- les copolymères greffés à base d'amidon tels que les Waterlock® (A-180 et G-400 par exemple) de chez Grain Processing Corporation,
- les acides polyacryliques de type SYNTHLEN K®,
- les copolymères acide polyacryliques acrylates d'alkyle de type PEMULEN®,
- l'AMPS (Acide polyacrylamidométhyl propane sulfonique neutralisé partiellement à l'ammoniaque et hautement réticulé) par exemple commercialisé par la société CARIANT,
- les copolymères AMPS/méthacrylates d'alkyle polyoxyéthylénés (réticulés ou non),
- le carboxy méthyl cellulose sodique et tous les dérivés ionisables de la cellulose, et
- leurs mélanges.

Conviennent tout particulièrement à l'invention le polyacrylate de sodium et le copolymère acrylamide/AMPS et leurs copolymères.

Il sera bien entendu fait en sorte que la teneur en polyélectrolyte soit ajustée de telle manière que l'aptitude à conférer un effet volumateur, et/ou un effet de gonflement lorsqu'il s'agit du maquillage des fibres kératiniques, soit effectivement manifeste.

Il est entendu que la quantité en polyélectrolyte est susceptible de varier significativement selon la nature du polyélectrolyte. De manière générale, cette quantité est au moins égale à la quantité nécessaire et suffisante pour conférer à ladite composition un effet volumateur. Elle est encore qualifiée de quantité efficace.

Selon une variante préférée, le polyélectrolyte est présent dans la composition cosmétique à phase continue huileuse de préférence dans une teneur en matière sèche allant de 0,05 à 40 % en poids, de façon encore plus préférée de 1 à 20 % en poids, et mieux encore de 5 à 10 % en poids par rapport au poids total de la composition.

Selon une autre variante, le polyélectrolyte est présent dans une teneur en poids supérieure ou égale à 0,05 %, et de façon préférée supérieure ou égale à 0,1 % et notamment supérieure ou égale à 0,5 % en poids, par rapport

au poids total de la composition cosmétique à phase continue huileuse.

Selon encore une autre variante, le polyélectrolyte est présent dans la composition cosmétique à phase continue huileuse de préférence dans une teneur allant de 0,05 à 15 % en poids, de façon encore plus préférée de 0,1 à 10 % en poids, et mieux encore de 0,5 à 5 % en poids par rapport au poids total de la composition à phase continue huileuse.

Sans que cela constitue une quelconque limitation à l'invention, les inventeurs ont émis l'hypothèse que le polyélectrolyte en dispersion dans l'huile joue le rôle de "pompe à eau". Ainsi, ce rôle de "pompe à eau" apparaît plus nettement lorsque la composition est mise en contact avec une phase aqueuse. Du fait de la présence de la pression osmotique élevée dans les réservoirs aqueux contenant le polyélectrolyte en raison de la présence de contre-ions, lesdits réservoirs gonflent.

Ainsi au niveau macroscopique, une fois que les deux compositions ont été déposées, c'est-à-dire mises en contact, par exemple au cours du maquillage, le film de la composition cosmétique conforme à l'invention, s'hydrate grâce aux nombreuses "pompes à eau" microdispersées dans le film. Ledit film gonfle et l'effet volumateur, et/ou l'effet de gonflement lorsqu'il s'agit du maquillage des fibres kératiniques, se manifeste.

## HUILES

La composition cosmétique comprise dans le kit de maquillage selon l'invention peut comprendre une ou plusieurs huiles ou solvant organique.

Ladite composition cosmétique à phase continue huileuse comprend habituellement une quantité d'huile totale variant de 10 à 90 %, de préférence de 15 à 80 % et mieux de 20 à 60 % en poids par rapport au poids total de la composition.

La ou les huiles présente(s) dans la composition de l'invention peu(ven)t être choisie(s) parmi les huiles volatiles et/ou les huiles non volatiles, et leurs mélanges.

On préfère, dans le cadre de la présente invention, les compositions cosmétiques comprenant essentiellement des huiles volatiles.

Par "huile ou solvant organique volatile", on entend une huile ou solvant organique (ou milieu non aqueux) susceptible de s'évaporer au contact de la peau en moins d'une heure, à température ambiante et pression atmosphérique.

L'huile volatile est une huile cosmétique volatile, liquide à température ambiante, ayant notamment une pression de vapeur non nulle, à température ambiante et pression atmosphérique, en particulier ayant une pression de valeur allant de 0,13 Pa à 40 000 Pa ($10^{-3}$ à 300 mm de Hg), et de préférence allant de 1,3 Pa à 8 000 Pa ( 0,01 à 60 mm de Hg).

Les huiles (ou solvants organiques) volatiles peuvent être des huiles hydrocarbonées, des huiles siliconées, des huiles fluorées ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux "d'Isopars® " ou de "Permetyls® ", les esters ramifiés en $C_8$-$C_{16}$, le néopentanoate d'isohexyle, et leurs mélanges. D'autres huiles hydrocarbonées volatiles comme les distillats de pétrole, notamment ceux vendus sous la dénomination "Shell Solt® " par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 6 centistokes ($6.10^{-6}$ $m^2/s$), et ayant notamment de 3 à 6 atomes de silicium, ces silicones comportant éventuellement un ou plusieurs groupes alkyles ou alkoxy ayant de 1 ou 2 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, l'heptaméthyl éthyl trisiloxane, l'heptaméthyl butyl trisiloxane, et leurs mélanges.

On peut également utiliser des solvants organiques volatils notamment fluorés tels que le nonafluorométhoxy butane ou le perfluorométhylcyclopentane.

Avantageusement, le ou les huile(s) volatile(s) est ou sont choisie(s) parmi les huiles volatiles hydrocarbonées ayant de 8 à 16 atomes de carbone, telle que l'isododécane, les huiles volatiles siliconées telles que le décaméthyl cyclopentasiloxane (D5), le dodécaméthyl cyclohexasiloxane (D6) et leurs mélanges.

La composition selon l'invention peut également comprendre au moins un composé non volatil, non soluble dans l'eau et liquide à température ambiante, notamment au moins une huile ou solvant organique non volatile, qui peut être en particulier choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces derniers pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société STÉARINERIES DUBOIS ou ceux vendus sous les dénominations de Miglyol 810® , 812® et 818® par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;

et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les huiles fluorées utilisables dans la composition de l'invention sont notamment des huiles fluorosiliconées, des polyéthers fluorés, des silicones fluorées telles que décrit dans le document EP-A-847752.

Le teneur en huile ou solvant organique volatile dans la composition cosmétique conforme à l'invention peut varier de 20 à 80 % en poids, en particulier de 30 à 70 % en poids, et mieux de 35 à 60 % en poids par rapport au poids total de la composition.

La teneur en huile ou solvant organique non volatile dans la composition cosmétique conforme à l'invention peut varier de 0,01 à 30 % en poids, en particulier de 0,1 à 25 % en poids, et mieux de 0,1 à 20 % en poids par rapport au poids total de la composition.

## COMPOSITION AQUEUSE

Au sens de l'invention, le terme "composition aqueuse" désigne une composition comprenant une quantité d'eau et/ou de solvant hydrosoluble tel que défini ci-après suffisante pour générer lors de sa mise en contact avec la composition cosmétique conforme à l'invention, un gonflement au niveau de cette composition en raison vraisemblablement du phénomène osmotique évoqué précédemment.

A titre de composition aqueuse, on peut utiliser toute composition aqueuse connue de l'homme de l'art.

Elle peut contenir un ou plusieurs polymère(s) susceptible(s) de former un film hydrophobe.

A ce titre, on peut se reporter aux polymères hydrophobes tels que définis ci-après.

Selon un mode de réalisation préféré, la composition aqueuse contient au moins 15 % en poids, de préférence au moins 20 % en poids et de façon encore plus préférée au moins 25 % en poids d'un polymère filmogène tel que décrit ci-après, par rapport au poids total de la composition aqueuse.

Il a en effet été constaté que dans ce mode de réalisation particulier, d'autant plus lorsque la composition aqueuse est déposée à la suite de la composition cosmétique conforme à l'invention, la présence de ce polymère filmogène permet de limiter l'évaporation en eau et donc améliore la pérennité de l'effet volumateur recherché.

Selon un autre mode de réalisation préféré, la composition aqueuse contient, au titre de polymère filmogène, un polymère en dispersion en phase aqueuse ou latex. On peut notamment citer les copolymères acrylates en émulsion à 40 %, tels que ceux vendus par la société Interpolymer sous la dénomination Syntran 5760® .

Avantageusement, le kit de maquillage et/ou de soin comporte alors une composition cosmétique à phase continue huileuse comportant également, à titre de polymère filmogène, un polymère en dispersion en phase aqueuse ou latex.

D'autres additifs peuvent être compris dans la composition aqueuse, comme cela est détaillé ci-après : gélifiants hydrosolubles, matières colorantes et charges. On cite ci-dessous quelques-uns des modes de réalisation préférentiels.

Ainsi, selon un mode de réalisation préféré, la composition aqueuse contient un gélifiant hydrosoluble, tel que détaillé ci-après, tel q'un gel aqueux de dérivé cellulosique, par exemple de l'hydroxyéthylcellulose.

Selon un autre mode de réalisation préféré de l'invention, la composition aqueuse peut contenir des charges. Comme cela apparaîtra ci-après, la composition aqueuse est de préférence transparente ou translucide. Pour cette raison, si la composition aqueuse contient des charges, ces dernières sont avantageusement elles-mêmes transparentes ou translucides, de sorte que la composition aqueuse conserve son caractère transparent ou translucide.

Selon un aspect de la présente invention, on préfère les kits dans lesquels la composition aqueuse est transparente ou translucide.

Par "transparent ou translucide" on entend la faculté à laisser passer la lumière sans provoquer de déviation par réfraction ou réflexion.

Plus particulièrement, on entend par "transparence ou translucidité", la faculté d'une épaisseur de 10 microns de la composition aqueuse à transmettre en moyenne au moins 25 % de la lumière dans la fenêtre de longueur d'onde de 400-700 nanomètres, de préférence 50 % de la lumière.

## TENSIOACTIF

La composition cosmétique conforme à l'invention contient au moins un agent tensioactif. possédant un HLB supérieur ou égal à 6 à 25 °C.

Selon un mode de réalisation particulier, il est ou ils sont présent(s) dans une teneur en poids supérieure ou égale à 0,1 % par rapport au poids total de la composition cosmétique conforme à l'invention. Il(s) peu(ven)t être présent(s) notamment en une teneur allant de 0,1 à 30 %, mieux de 0,5 à 15 % et mieux de 1,5 à 10 % en poids par rapport au poids total de la composition cosmétique.

Il est entendu que lorsque le polyélectrolyte est incorporé à la composition conforme à l'invention sous forme d'une composition déjà formulée avec un agent tensioactif, la quantité en agent tensioactif ci-dessus définie tient compte de la teneur en ledit agent tensioactif comprise dans la formulation du polyélectrolyte.

Le "HLB supérieur ou égal à 6" s'entend d'un tensioactif possédant à 25 °C une balance HLB (hydrophile-lipophile-balance) au sens de GRIFFIN supérieure ou égale à 6.

La valeur HLB selon GRIFFIN est définie dans J. Soc. Cosm. Chem. 1954 (volume 5), pages 249-256.

On peut se reporter au document "Encyclopedia of Chemical Technology, KIRK-OTHMER", volume 22, p. 333-432, 3ème édition, 1979, WILEY, pour la définition des propriétés et des fonctions (émulsionnant) des tensioactifs, en particulier p. 347-377 de cette référence, pour les tensioactifs non ioniques.

L'agent tensioactif compris dans la composition cosmétique comprise dans le kit selon la présente invention peut être ionique, non ionique ou à caractère mixte ionique et non ionique.

Parmi les agents tensioactifs non ioniques pouvant être présents dans la composition à phase continue huileuse, utilisés seuls ou en mélange; on peut citer notamment :

- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool cétéarylique à 30 groupes oxyéthylénés (nom CTFA "Ceteareth-30 ") et l'éther oxyéthyléné du mélange d'alcools gras en $C_{12}$-$C_{15}$ comportant 7 groupes oxyéthylénés (nom CTFA "$C_{12}$-$_{15}$ Pareth-7") par exemple commercialisé sous la dénomination NEODOL 25-7® par SHELL CHEMICALS,
- les esters d'acide gras (notamment d'acide en $C_8$-$C_{24}$, et de préférence en $C_{16}$-$C_{22}$) et de polyéthylène glycol (pouvant comprendre de 1 à 150 motifs d'éthylèneglycol) tels que le stéarate de PEG-50 et le monostéarate de PEG-40 par exemple commercialisé sous le nom MYRJ 52P® par la société ICI UNIQUEMA,
- les esters d'acide gras (notamment d'acide en $C_8$-$C_{24}$, et de préférence en $C_{16}$-$C_{22}$) et des éthers de glycérol oxyéthylénés et/ou oxypropylénés (pouvant comporter de 1 à 150 groupes oxyéthylénés et/ou oxypropylénés), comme le monostéarate de PEG-200 glycéryle par exemple vendu sous la dénomination Simulsol 220 TM® par la société SEPPIC ; le stéarate de glycéryle polyéthoxylé à 30 groupes d'oxyde d'éthylène comme le produit TAGAT S® vendu par la société GOLDSCHMIDT, l'oléate de glycéryle polyéthoxylé à 30 groupes d'oxyde d'éthylène comme le produit TAGAT O® vendu par la société GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle

polyéthoxylé à 30 groupes d'oxyde d'éthylène comme le produit TAGAT L® vendu par la société GOLDSCHMIDT et le laurate de glycéryle polyéthoxylé à 30 groupes d'oxyde d'éthylène comme le produit TAGAT I® de la société GOLDSCHMIDT,

- les esters d'acide gras (notamment d'acide en $C_8$-$C_{24}$, et de préférence en $C_{16}$-$C_{22}$) et des éthers de sorbitol oxyéthylénés et/ou oxypropylénés (pouvant comporter de 1 à 150 groupes oxyéthylénés et/ou oxypropylénés), comme le polysorbate 60 par exemple vendu sous la dénomination Tween 60® par la société UNIQUEMA, ainsi que le polysorbate 80, le polysorbate 40 et le polysorbate 20 .
- la diméthicone copolyol, telle que celle vendue sous la dénomination Q2-5220® par la société DOW CORNING,
- la diméthicone copolyol benzoate (FINSOLV SLB 101® et 201® de la société FINTEX),
- les copolymères d'oxyde propylène et d'oxyde d'éthylène, également appelés polycondensats OE/OP,
- et leurs mélanges.

Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

formule dans laquelle a va de 2 à 120, et b va de 1 à 100.

Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol par exemple vendus sous les dénominations SYNPERONIC® comme les SYNPERONIC PE/ L44® et SYNPERONIC PE/F127® par la société ICI.

Parmi les agents tensioactifs ioniques, qui peuvent être anioniques ou cationiques, pouvant être présents dans la composition à phase continue huileuse, utilisés seuls ou en mélange, on peut citer notamment :

- les tensioactifs siliconés comme les diméthicones copolyols phosphates tels que celui vendu sous la dénomination PECOSIL PS 100® par la société PHOENIX CHEMICAL,
- les dérivés d'acide aminés, tels que le lauryl sarcosinate, et le lauryl taurate,
- les sels d'acides gras en $C_{16}$-$C_{30}$, notamment ceux dérivant des amines, comme le stéarate de triéthanolamine,
- les sels d'acides gras polyoxyéthylénés, notamment ceux dérivant des amines ou les sels alcalins, et leurs mélanges,
- les esters phosphoriques et leurs sels tels que le "DEA oleth-10 phosphate" (Crofados N 10N® de la société CRODA),
- les sulfosuccinates tels que le "Disodium PEG-5 citrate lauryl sulfosuccinate" et le "Disodium ricinoleamido MEA sulfosuccinate",
- les alkyléthersulfates tels que le lauryl éther sulfate de sodium,
- les iséthionates,
- les acylglutamates tels que le "Disodium hydrogenated tallow glutamate" (AMISOFT HS-21 R® commercialisé par la société AJINOMOTO) et leurs mélanges.

Convient tout particulièrement à l'invention le stéarate de triéthanolamine. Ce dernier est généralement obtenu par simple mélange de l'acide stéarique et de la triéthanolamine.

A titre représentatif des tensioactifs cationiques, on peut notamment citer :

- les alkyl-imidazolidinium tels que l'étho-sulfate d'isostéaryl-éthylimidonium,
- Les sels d'ammonium tels que le chlorure de N,N,N,-triméthyl-1-docosanamimium (chlorure de Behentrimonium).

La composition à phase continue huileuse peut également contenir un ou plusieurs tensioactifs amphotériques comme les N-acyl-aminoacides, tels que les N-alkyl-aminoacétates et le cocoamphodiacétate.

Il sera bien entendu fait en sorte que l'agent tensioactif soit choisi de telle façon et soit présent dans une quantité telle que la composition cosmétique conforme à l'invention se présente bien sous forme d'une émulsion à phase continue huileuse.

Par ailleurs, la composition cosmétique conforme à la présente invention comprent de préférence un ou des agent(s) tensioactif(s) de HLB supérieur ou égal à 8 à 25 °C, et de façon encore plus préférée de HLB supérieur ou égal à 10, ou encore supérieur ou égal à 12.

La composition cosmétique conforme à l'invention, dans la mesure où il s'agit d'une composition à phase continue huileuse peut contenir également un agent tensioactif de bas HLB, à savoir inférieur à 6 parmi lesquels on

peut citer :

a) les agents tensioactifs non ioniques de HLB inférieur à 6 à 25 °C, tels que :

- les esters et éthers d'oses tels que les stéarate de sucrose, cocoate de sucrose, stéarate de sorbitan et leurs mélanges comme l'Arlatone 2121® commercialisé par la société ICI ;
- les esters d'acides gras (notamment d'acide en $C_8$-$C_{24}$, et de préférence en $C_{16}$-$C_{22}$) et de polyol, notamment de glycérol ou de sorbitol, tels que stéarate de glycéryle, stéarate de glycéryle tel que le produit vendu sous la dénomination TEGIN M® par la société GOLDSCHMIDT, laurate de glycéryle tel que le produit vendu sous la dénomination IMWITOR 312® par la société HULS, stéarate de polyglycéryl-2, tristéarate de sorbitan, ricinoléate de glycéryle ;
- le mélange de cyclométhicone/diméthicone copolyol vendu sous la dénomination Q2-3225C® par la société DOW CORNING,
- les alcools gras tels que l'alcool cétylique, l'alcool stéarique,
- les alcools gras éthoxylés avec un nombre d'éthoxylation tel que le HLB est inférieur à 6.

Conviennent tout particulièrement à l'invention, à titre d'agent tensioactif de HLB supérieur ou égal à 6 à 25 °C, les esters d'acide gras et des éthers de sorbitol oxyéthylénés et/ou oxypropylénés (pouvant comporter de 1 à 150 groupes oxyéthylénés) comme le polysorbate 20 de HLB 16,7, le polysorbate 40 de HLB 15,6, le polysorbate 60 de HLB 14,9 et le polysorbate 80 de HLB 15,0.

La composition cosmétique conforme à l'invention peut comprendre d'autre(s) tensioactif(s) qui sont par exemple introduits dans la composition par l'introduction de la dispersion aqueuse de particules d'un polymère, ces tensioactifs étant ceux classiquement utilisés pour les stabiliser.

**<u>AGENT STRUCTURANT</u>**

La composition cosmétique comprise dans le kit de maquillage selon l'invention peut comprendre au moins un agent structurant de la phase huileuse ou solvant organique choisi parmi les cires, les polymères semi-cristallins, les gélifiants lipophiles et leurs mélanges.

L'agent structurant peut représenter de 0,1 à 80 % en poids par rapport au poids total de la composition cosmétique, de préférence de 0,5 à 50 % et de façon encore plus préférée de 1 à 40 % en poids.
La quantité en structurant huileux peut être ajustée par l'homme du métier en fonction du propriétés de structuration desdits agents.

<u>Cire(s)</u>

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination "MDSC 2920" par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions cosmétiques comprises dans le kit de maquillage selon l'invention sont choisies parmi les cires, solides, à température ambiante d'origine animale, végétale, minérale ou

de synthèse et leurs mélanges.

Les cires pouvant être utilisées dans les compositions cosmétiques présentent généralement une dureté allant de 0,01 MPa à 15 MPa, notamment supérieure à 0,05 MPa et en particulier supérieure à 0,1 MPa.

La dureté est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.

Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.

La valeur de la dureté est la force de compression maximale mesurée divisée par la surface du cylindre du texturomètre en contact avec la cire.

A titre illustratif des cires convenant à l'invention, on peut notamment citer les cires hydrocarbonées comme la cire d'abeilles, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50® , l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone, les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.

Selon un mode de réalisation particulier, les compositions cosmétiques comprises dans le kit de maquillage selon l'invention peuvent comprendre au moins une cire dite cire collante c'est-à-dire possédant un collant supérieur ou égal à 0,1 N.s et une dureté inférieure ou égale à 3,5 MPa.

La cire collante utilisée peut posséder notamment un collant allant de 0,1 N.s à 10 N.s, en, particulier allant de 0,1 N.s à 5 N.s, de préférence allant de 0,2 à 5 N.s et mieux allant de 0,3 à 2 N.s.

Le collant de la cire est déterminé par la mesure de l'évolution de la force (force de compression) en fonction du temps, à 20 °C selon le protocole indiqué précédemment pour la dureté.

Pendant le temps de relaxation de 1 s, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force. La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa.

Comme cire collante, on peut utiliser un (hydroxystéaryloxy)stéarate d'alkyle en $C_{20}$-$C_{40}$ (le groupe alkyle comprenant de 20 à 40 atomes de carbone), seul ou en mélange

Une telle cire est notamment vendue sous les dénominations « Kester Wax K 82 P® » et « Kester Wax K 80 P® » par la société KOSTER KEUNEN.

Les cires citées ci-dessus présentent généralement un point de fusion commençante inférieur à 45 °C.

Dans la présente invention, on peut également utiliser des cires fournies sous forme de petites particules ayant une dimension exprimée en diamètre "effectif" moyen en volume D[4,3] de l'ordre de 0,5 à 30 micromètres, en particulier de 1 à 20 micromètres, et plus particulièrement de 5 à 10 micromètres, désignées par la suite par l'expression "micro cires". A des fins de distinction, les cires mises en oeuvre selon l'invention sous forme de fragments de dimension plus élevée sont par la suite désignées par l'expression "cires de type traditionnel".

Les tailles de particules peuvent être mesurées par différentes techniques, on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions cosmétiques, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre "effectif" de particules. Cette théorie est notamment décrite dans l'ouvrage de Van de Hulst, H.C., "Light Scattering by Small Particles," Chapitres 9 et 10, Wiley, New York, 1957.

La composition cosmétiques est caractérisée par son diamètre "effectif" moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Les mesures sont réalisées à 25 °C, sur une dispersion de particules diluée, obtenue à partir de la composition cosmétique de la manière suivante : 1) dilution d'un facteur 100 avec de l'eau, 2) homogénéisation de la solution, 3) repos de la solution durant 18 heures, 4) récupération du surnageant homogène blanchâtre.

Le diamètre "effectif" est obtenu en prenant un indice de réfraction de 1,33 pour l'eau et un indice de réfraction moyen de 1,42 pour les particules.

Comme micro cires pouvant être utilisées dans les compositions cosmétiques conformes à l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200® , 220® , 220L® et 250S® par la société MICRO POWDERS et les micro cires de polytétrafluoroéthylène telles que celles commercialisées sous les dénominations de Microslip 519® et 519 L® par la société MICRO POWDERS.

Dans la composition cosmétique comprise dans le kit de maquillage selon l'invention, on peut bien entendu utiliser un mélange de cires et notamment utiliser une ou plusieurs cires de type traditionnel telles que notamment une cire collante et une ou plusieurs cires dites micro cires. Ladite composition cosmétique peut comprendre une teneur en cires allant de 0,1 à 70 % en poids par rapport au poids total de la composition cosmétique, en particulier elle peut en contenir de 0,5 à 50 %, plus particulièrement de 1 à 30%.

Polymères semi-cristallins

On entend par polymère des composés comportant au moins deux motifs, de préférence au moins 3 motifs et plus spécialement au moins 10 motifs de répétition. Par "polymère semi-cristallin", on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin a une température de fusion supérieure ou égale à 30 °C (notamment allant de 30 °C à 80 °C), de préférence allant de 30 °C à 60 °C. Cette température de fusion est une température de changement d'état du premier ordre.

Cette température de fusion peut être mesurée par toute méthode connue et en particulier à l'aide d'un calorimètre à balayage différentiel (D.S.C).

De façon avantageuse, le ou les polymères semi-cristallins auxquelles s'applique l'invention présentent une masse moléculaire moyenne en nombre supérieure ou égale à 1 000. De façon avantageuse, le ou les polymères semi-cristallins de la composition cosmétique conforme à l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être chaîne comportant au moins 6 atomes de carbone.

Le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

De tels polymères sont décrits par exemple dans le document EP 1396259.

*A. <u>Polymères semi-cristallins à chaînes latérales cristallisables</u>*

On peut citer en particulier ceux définis dans le document US-A-5,156,911 et WO-A-01/19333. Ce sont des homopolymères ou copolymères comportant de 50 à 100 % en poids de motifs résultant de la polymérisation d'un ou plusieurs monomères porteurs de chaîne latérale hydrophobe cristallisable.

Ces homo- ou co-polymères sont de toute nature du moment qu'ils présentent les conditions indiquées précédemment.

*B. <u>Les polymères portant dans le squelette au moins une séquence cristallisable</u>*

Ces polymères sont notamment des copolymères séquencés constitués d'au moins 2 séquences de nature chimique différente dont l'une est cristallisable.

- On peut utiliser les polymères séquencés définis dans le brevet US-A-5,156,911 ;
- Les copolymères séquencés d'oléfine ou de cyclooléfine à chaîne cristallisable comme ceux issus de la polymérisation séquencée de :
- cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
- avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
- et en particulier les copoly(éthylène/norbornène) blocs et les terpolymères (éthylène/propylène/éthylidène-norbomène) blocs. On peut aussi utiliser ceux résultants de la copolymérisation séquencée d'au moins 2 $\alpha$-oléfines en $C_2$-$C_{16}$ et mieux en $C_2$-$C_{12}$ et encore mieux en $C_4$-$C_{12}$ tels que ceux cités précédemment et en particulier les bipolymères séquencés d'éthylène et d'1-octène.
- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
- Séquence cristallisable par nature : a) polyester comme les poly(alkylène téréphtalate), b) polyoléfine comme les polyéthylènes ou polypropylènes.
- Séquence amorphe et lipophile comme les polyoléfines ou copoly(oléfme)s amorphes telles que le poly(isobutylène), le polybutadiène hydrogéné, le poly(isoprène) hydrogéné.

Comme exemple de tels copolymères à séquence cristallisable et à séquence amorphe distinctes, on peut citer :

$\alpha$) les copolymères séquencés poly($\varepsilon$-caprolactone)-b-poly(butadiène), utilisés de préférence hydrogénés, tels que ceux décrits dans l'article "Melting behavior of poly($\varepsilon$-caprolactone)-block-polybutadiène copolymers" de S. Nojima, Macromolécules, 32, 3727-3734 (1999).

$\beta$) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al.,

Polymer Bulletin, 34, 117-123 (1995).

γ) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et "Polymer agregates with crystalline cores : the system poly(ethylene)-poly(ethylene-propylene)" de P. Richter et al., Macromolécules, 30, 1053-1068 (1997).

δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

De préférence, les polymères semi-cristallins de la composition cosmétique conforme à l'invention sont non réticulés.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en $C_{14}$ à $C_{24}$, les (méth)acrylates de perfluoroalkyle en $C_{11}$ à $C_{15}$, les N alkyl (méth)acrylamides en $C_{14}$ à $C_{24}$ avec ou sans atome de fluor, les esters vinyliques à chaînes alkyle ou perfluoroalkyle en $C_{14}$ à $C_{24}$, les éthers vinyliques à chaînes alkyle ou perfluoroalkyle en $C_{14}$ à $C_{24}$, les alphaoléfmes en $C_{14}$ à $C_{24}$, les para-alkyl styrènes avec un groupe alkyle comportant de 12 à 24 atomes de carbone, avec au moins un ester ou amide d'acide monocarboxylique en $C_1$ à $C_{10}$ éventuellement fluoré, qui peut être représenté par la formule suivante :

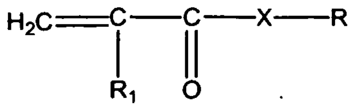

dans laquelle $R_1$ est H ou $CH_3$, R représente un groupe alkyle en $C_1$-$C_{10}$ éventuellement fluoré et X représente O, NH ou $NR_2$, où $R_2$ représente un groupe alkyle en $C_1$-$C_{10}$ éventuellement fluoré.

Selon un mode plus particulier de réalisation de l'invention, le polymère est issu d'un monomère à chaîne cristallisable choisi parmi les (méth)acrylates d'alkyle saturés en $C_{14}$ à $C_{22}$.

A titre d'exemple particulier de polymère semi-cristallin structurant utilisable dans la composition cosmétique, on peut citer les produits Intelimer® de la société Landec décrits dans la brochure "Intelimer® polymers", Landec IP22 (Rev. 4-97). Ces polymères sont sous forme solide à température ambiante (25 °C). Ils sont porteurs de chaînes latérales cristallisables et présentent la formule X précédente.

*Gélifiants lipophiles*

Les gélifiants utilisables dans les compositions cosmétiques comprises dans les kits de maquillage selon l'invention peuvent être des gélifiant lipophiles organiques ou minéraux, polymériques ou moléculaires.

Comme gélifiant lipophile minéral, on peut citer les argiles éventuellement modifiées comme les hectorites modifiées par un chlorure d'ammonium d'acide gras en $C_{10}$ à $C_{22}$, comme l'hectorite modifiée par du chlorure de distéaryl di-méthyl ammonium telle que, par exemple, celle commercialisée sous la dénomination de Bentone 38V® par la société ELEMENTIS.

On peut également citer la silice pyrogénée éventuellement traitée hydrophobe en surface dont la taille des particules est inférieure à 1 μm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références Aerosil R812® par la société DEGUSSA, CAB-O-SIL TS-530® par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références Aerosil R972® , et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à

micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6® , KSG16® et de KSG18® par la société SHIN-ETSU, de Trefil E-505C® et Trefil E-506C® par la société DOW-CORNING, de Gransil SR-CYC® , SR DMF10® , SR-DC556® , SR 5CYC gel® , SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les polycondensats de type polyamide résultant de la condensation entre ($\alpha$) au moins un acide choisi parmi les acides dicarboxyliques comprenant au moins 32 atomes de carbone tels que les acides gras dimères et ($\beta$) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges. Les copolymères séquencés de type "dibloc", "tribloc" ou "radial" du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans les compositions cosmétiques comprises dans les kits de maquillage selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

## POLYMERE FILMOGENE

La composition cosmétique à phase continue huileuse, ainsi que la composition aqueuse, comprises dans le kit de maquillage selon l'invention peuvent comprendre selon un mode de réalisation particulier au moins un polymère filmogène.

Le polymère filmogène peut être présent dans la composition cosmétique à phase continue huileuse en une teneur en matières sèches allant de 0,1 % à 60 % en poids par rapport au poids total de la composition cosmétique, de préférence de 0,5 % à 40 % en poids, et mieux de 10 % à 30 % en poids.

Les teneurs en polymères filmogènes propres à la composition aqueuse ont déjà été indiquées plus haut dans le paragraphe "composition aqueuse".

Dans la présente invention, on entend par "polymère filmogène", un polymère apte à former à lui seul ou en présence d'un agent auxiliaire de filmification, un film macroscopiquement continu et adhérent sur les fibres kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.

Parmi les polymères filmogènes utilisables dans la composition cosmétique à phase continue huileuse et la composition aqueuse comprises dans le kit selon l'invention, on peut citer les polymères synthétiques, de type radicalaire ou de type polycondensat, les polymères d'origine naturelle, et leurs mélanges.

Ces polymères filmogènes sont de préférence distincts du polyélectrolyte précédemment défmi.

Les polymères liposolubles et les polymères dispersés en phase non aqueuse sont de préférence compris dans la composition à phase continue huileuse, alors que les polymères liposolubles et les polymères sous forme dispersée en phase aqueuse, peuvent être compris à la fois dans la composition à phase continue huileuse et dans la composition aqueuse.

### *Polymères liposolubles*

Selon une variante de réalisation, la composition cosmétique comprise dans le kit de maquillage selon l'invention comprend un polymère filmogène pouvant être un polymère solubilisé dans la phase huileuse comprenant des huiles ou solvants organiques tels que ceux décrits précédemment (on dit alors que le polymère filmogène est un polymère liposoluble).

A titre d'exemple de polymère liposoluble, on peut citer les copolymères d'ester vinylique (le groupe vinylique

étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2000 à 500 000 et de préférence de 4000 à 200 000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

A titre d'exemples de résines polymethylsilsesquioxanes commercialement disponibles, on peut citer celles qui sont commercialisés :

- par la société Wacker sous la référence Resin MK tels que la Belsil PMS MK:
- par la société SHIN-ETSU sous les références KR-220L.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.

On peut également utiliser les polyamides siliconés du type polyorganosiloxane tels que ceux décrits dans les documents US-A-5 874 069, US-A-5,919,441, US-A-6,051,216 et US-A-5,981,680.

Ces polymères siliconés peuvent appartenir aux deux familles suivantes :

1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1 411 069 ou WO 04/028488.

*Polymères hydrosolubles*

Selon une autre variante de réalisation, la composition cosmétique à phase continue huileuse et la composition aqueuse comprises dans le kit de maquillage selon l'invention, comprennent au moins un polymère filmogène pouvant être un polymère hydrosoluble. Le polymère est alors solubilisé dans la phase aqueuse de la composition. Comme exemples de polymères filmogènes hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :

- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels les chondroïtines sulfate,

et leurs mélanges.

*Polymères d'origine naturelle*

Les polymères d'origine naturelle, éventuellement modifiés, peuvent être choisis parmi la résine shellac, la gomme de sandaraque, les dammars, les élémis, les copals, les polymères cellulosiques, et leurs mélanges.

*Polymères sous forme dispersée*

Le polymère filmogène peut être présent sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

a) Dispersion aqueuse

Comme dispersion aqueuse de polymère filmogène, on peut utiliser les dispersions acryliques vendues sous les dénominations Neocryl XK-90® , Neocryl A-1070® , Neocryl A-1090® , Neocryl BT-62® , Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer Allianz Opt® par la société Rohm and Haas ou encore les dispersions aqueuses de polyuréthane vendues sous les dénominations Neorez R-981® et Neorez R-974® par la société AVECIA-NEORESINS, les Avalure UR-405® , Avalure UR-410® , Avalure UR-425® , Avalure UR-450® , Sancure 875® , Avalure UR-445® et Sancure 2060® par la société NOVEON, Impranil 85® par la société BAYER, Aquamere H-1511® par la société HYDROMER ; les sulfopolyesters vendus sous le nom de marque Eastman AQ® par la société EASTMAN CHEMICAL PRODUCTS, les dispersions vinyliques comme le Mexomère PAM® , les dispersions aqueuses de polyvinyl acétate comme le "Vinybran® " de la société Nisshin Chemical

ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références "Hybridur® " par la société AIR PRODUCTS ou "Duromer® " de NATIONAL STARCH, les dispersions type core/shell : par exemple celles commercialisées par la société ATOFINA sous la référence Kynar (core : fluoré- shell : acrylique) ou encore ceux décrits dans le document US 5 188 899 (core ; silice - shell : silicone) et leurs mélanges.

b) Dispersion non aqueuse

On peut aussi citer les dispersions de particules d'un polymère éthylénique greffé, de préférence acrylique, dans une phase huileuse liquide, le polymère éthylénqiue étant avantageusement dispersé en l'absence de stabilisant additionnel en surface des particules telles que décrite notamment dans le document WO 04/055081.

La composition cosmétique conforme à l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

A ce titre, on peut citer les dispersions acryliques dans l'isododécane comme le Mexomère PAP® par la société CHIMEX.

Selon un mode de réalisation privilégié de la présente invention, la composition cosmétique à phase continue huileuse comprise dans le kit de maquillage comprend au moins 10 % en poids en matière sèche d'au moins un polymère filmogène, de préférence, au moins 15 % en poids et encore mieux au moins 20 % en poids par rapport au poids total de la composition.

Selon un mode de réalisation avantageux de la présente invention, la composition aqueuse comprise dans le kit de maquillage comprend au moins 10 % en poids en matière sèche d'une dispersion aqueuse d'au moins un polymère filmogène de préférence, au moins 15 % en poids par rapport au poids total de la composition.

## EAU ET/OU SOLVANT HYDROSOLUBLE

Il est à noter que les compositions cosmétiques et aqueuses comprises dans le kit de maquillage selon l'invention peuvent comprendre indépendamment l'une de l'autre une phase aqueuse comprenant de l'eau et/ou au moins un solvant hydrosoluble. Bien entendu, concernant la composition cosmétique à phase continue huileuse, cette quantité de phase aqueuse est ajustée pour préserver une marge de gonflement des réservoirs aqueux de polyélectrolytes suffisants pour que l'effet volumateur soit manifeste en terme d'effet de maquillage, lors de la mise en contact des deux compositions, par exemple au cours du maquillage.

Par "solvant hydrosoluble", on désigne dans la présente invention un composé liquide à température ambiante et miscible à l'eau (miscibilité dans l'eau supérieure à 50 % en poids à 25 °C et pression atmosphérique).

Parmi les solvants hydrosolubles pouvant être utilisés dans lesdites compositions cosmétiques, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en $C_3$ et $C_4$ et les aldéhydes en $C_2$-$C_4$.

La phase aqueuse (eau et/ou solvant(s) hydrosoluble(s)) peut être introduite en tant que telle dans les compositions cosmétique et aqueuse comprises dans le kit de maquillage ou y être incorporée par le biais, d'un ou plusieurs ingrédients constituant lesdites compositions cosmétique et aqueuse. Ainsi de l'eau peut être notamment introduite dans la composition cosmétique par le biais de l'introduction de latex ou de pseudolatex, c'est-à-dire de dispersion aqueuse de particules de polymère, ou encore de latex inverse, tel que les différents types de Simulgel® .

La teneur en eau et/ou en solvant(s) hydrosoluble(s) dans la composition cosmétique à phase continue huileuse comprise dans le kit de maquillage selon l'invention est de préférence inférieure à 30 % en poids, de préférence inférieure à 25 %, ou encore à 20 % et notamment à 15 % en poids par rapport au poids total de la composition cosmétique.

Outre les ingrédients précités, chacune des compositions cosmétique ou aqueuse comprises dans le kit de maquillage selon l'invention peut comprendre d'autres additifs classiques dans le domaine de la cosmétique sous réserve que leurs quantités respectives ne portent pas préjudice à la manifestation de l'effet volumateur recherché au niveau du maquillage final.

## GELIFIANT HYDROSOLUBLE

La composition aqueuse comprise dans le kit de maquillage selon l'invention peut comprendre un gélifiant hydrosoluble.

Les polymères filmogènes hydrosolubles cités plus haut peuvent également jouer le rôle de gélifiant hydrosoluble. A ce titre, on peut par exemple citer l'hydroxyéthylcellulose.

Le polymère gélifiant hydrosoluble peut être présent dans la composition aqueuse en une teneur en matière sèche allant de 0,01 % à 60 % en poids, de préférence de 0,25 % à 40 % en poids, mieux de 0,5 % à 30 % en poids, voire de 1 à 20 % en poids par rapport au poids total ou de la composition aqueuse.

## MATIÈRE COLORANTE

Les compositions cosmétique et aqueuse comprises dans les kits de maquillage selon l'invention peuvent indépendamment l'une de l'autre également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30 % en poids par rapport au poids total de ladite composition cosmétique ou de la composition aqueuse.

## CHARGES

Les compositions cosmétique et aqueuse comprises dans les kits de maquillage selon l'invention peuvent indépendamment l'une de l'autre également comprendre au moins une charge.

Les charges peuvent être choisies parmi celles bien connues de l'homme du métier et couramment utilisées dans les compositions cosmétiques et aqueuses. Les charges peuvent être minérales ou organiques, lamellaires ou sphériques. On peut citer le talc, le mica, la silice, le kaolin, les poudres de polyamide comme le Nylon® commercialisé sous la dénomination Orgasol® par la société ATOCHEM, de poly-β-alanine et de polyéthylène, les poudres de polymères de tétrafluoroéthylène comme le Téflon® , la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel® par la société NOBEL INDUSTRIE, les poudres acryliques telles que celles commercialisées sous la dénomination Polytrap® par la société DOW CORNING, les particules de polyméthacrylate de méthyle et les microbilles de résine de silicone (Tospearls® de TOSHIBA, par exemple), le carbonate de calcium précipité, le carbonate et l'hydro-carbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

On peut également utiliser un composé susceptibles de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel® 820 DU 40 et Expancel® 007WU par la Société AKZO NOBEL.

Les charges peuvent représenter de 0,1 à 25 %, en particulier de 1 à 20 % en poids par rapport au poids total de la composition cosmétique ou de la composition aqueuse.

Les compositions cosmétique et aqueuse comprises dans les kits de maquillage selon l'invention peuvent indépendamment l'une de l'autre également comprendre, en outre, tout additif usuellement utilisé en cosmétique tels que les antioxydants, les conservateurs, les fibres, les parfums, les neutralisants, les vitamines, les agents de coalescence, les plastifiants, et leurs mélanges.

## FIBRES

Les compositions cosmétique et aqueuse comprises dans les kits de maquillage selon l'invention peuvent indépendamment l'une de l'autre également en outre comprendre des fibres qui permettent notamment une amélioration de l'effet allongeant dans le domaine du maquillage des fibres kératiniques.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans les compositions comprises dans les kits de maquillage de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 $\mu$m à 10 mm, en particulier de 0,1 mm à 5 mm et plus particulièrement de 0,3 mm à 3,5 mm. Leur section peut être comprise dans un cercle de diamètre allant de 2 nm à 500 $\mu$m, en particulier allant de 100 nm à 100 $\mu$m et plus particulièrement de 1 $\mu$m à 50 $\mu$m. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans les compositions comprises dans les kits de maquillage selon l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans les compositions comprises dans les kits de maquillage selon l'invention, on peut citer les fibres non rigides telles que les fibres de polyamide (Nylon® ) ou les fibres rigides telles que les fibres de polyimide-amide comme celles vendues sous les dénomination KERMEL® , KERMEL TECH® par la société RHODIA ou de poly-(p-phénylène-téréphtalamide) (ou d'aramide) notamment vendues sous la dénomination Kevlar® par la société DUPONT DE NEMOURS.

Les fibres peuvent êtres présentes dans la composition cosmétique ou la composition aqueuse en une teneur allant de 0,01 % à 10 % en poids, par rapport au poids total de la composition cosmétique ou de la composition aqueuse, en particulier de 0,05 % à 5 % en poids, et plus particulièrement de 0,1 % à 3 % en poids.

## ACTIFS COSMÉTIQUES

Les compositions cosmétique et aqueuse comprises dans les kits de maquillage selon l'invention peuvent indépendamment l'une de l'autre également, de plus, comprendre tous les ingrédients classiquement utilisés en cosmétique. Ces ingrédients peuvent notamment être choisis parmi les polymères, notamment les polymères fixants ; les agents conditionneurs de cheveux ; les opacifiants ; les parfums ; les épaississants ; les gélifiants ; les colorants capillaires ; les résines de silicone ; les gomme de silicone ; les conservateurs ; les antioxydants, les actifs cosmétiques ; les filtres solaires ; les agents de stabilisation du pH ; les vitamines ; les hydratants ; les agents antitranspirants ; les agents déodorants ; les composés autobronzants et leurs mélanges. Les quantités de ces différents ingrédients sont celles classiquement utilisées dans les domaines concernés et par exemple de 0,01 à 20 % en poids par rapport au poids total de la composition cosmétique et de 0,1 à 10 % en poids par rapport au poids total de la composition aqueuse.

Bien entendu, l'homme du métier veillera à choisir les éventuels additifs complémentaires et/ou leur quantité de telle manière que les propriétés avantageuses desdites compositions cosmétique et aqueuse ne soient pas, ou substantiellement pas, altérées par l'adjonction envisagée.

## FORMULATION

La composition cosmétique comprise dans le kit de maquillage selon l'invention peut se présenter sous forme liquide, pâteuse, solide, de mousse ou de spray.

Le kit selon l'invention peut être utilisé pour le maquillage de la peau, des lèvres et/ou des fibres kératiniques d'être humain. Le kit trouve donc une application particulière comme kit de maquillage du corps ou du visage tel que fond de teint, rouge à lèvres, soin pour lèvres, vernis à ongles, soin des ongles, mascara, eye-liner ; kit capillaire tel que kit de coloration ou de maintien de coiffure des cheveux ou encore kit de protection solaire.

Selon un aspect préféré de l'invention, le kit est sous la forme de rouges à lèvres ou de produits du teint, notamment du type fond de teint, ou encore de mascara.

Selon un mode de réalisation particulier, à savoir lorsque la composition aqueuse est appliquée en second lieu, les compositions aqueuses comprises dans le kit de maquillage selon l'invention peuvent se présenter sous toute forme adaptée à une application de type "top coat". Ainsi que cela est exposé ci-dessus, elles peuvent avantageusement prendre la forme d'un gel.

## PROCÉDÉ DE PREPARATION

Dans tous les cas, les compositions cosmétiques et les compositions aqueuses comprises dans le kit de maquillage selon l'invention peuvent être préparées selon des méthodes connues de l'homme du métier.
Dans le cas d'un kit destiné au maquillage des fibres kératiniques, le procédé de préparation des compositions selon l'invention dépend du type de mascara recherché.

## PROCEDE DE MAQUILLAGE ET/OU DE SOIN NON THERAPEUTIQUE

La présente invention concerne un procédé cosmétique de maquillage et/ou de soin non thérapeutique de la peau, des lèvres et/ou des fibres kératiniques comprenant au moins un étape d'application sur la peau, les lèvres et/ou les fibres kératiniques d'une composition cosmétique telle que défmie précédemment et au moins une étape d'application d'une composition aqueuse.
La présente invention a encore pour objet un procédé de maquillage et/ou de soin non thérapeutique des fibres kératiniques, comprenant au moins une étape d'application sur lesdites fibres kératiniques notamment les cils, d'une composition cosmétique telle que définie précédemment et au moins une étape d'application d'une composition aqueuse. Les deux étapes peuvent avantageusement être effectuées dans l'ordre préférentiel de l'application de la composition cosmétique suivie de l'étape d'application de la composition aqueuse. De cette façon, la manifestation de l'effet volumateur ou de gonflement des cils est optimale.
Le kit selon la présente invention peut avantageusement comprendre un ou plusieurs moyens d'application des compositions cosmétiques et aqueuses sur la surface à maquiller.
Les compositions cosmétiques et les compositions aqueuses comprises dans le kit de maquillage de l'invention, lorsqu'elles sont destinées au maquillage des fibres kératiniques, peuvent être en particulier appliquées sur les cils, à l'aide d'une brosse ou d'un peigne.
L'effet épaississant du maquillage peut par ailleurs être renforcé en sélectionnant tout particulièrement le moyen d'application successive des compositions cosmétiques et aqueuses, qui pourra être en particulier une brosse de maquillage.
En l'occurrence, il est particulièrement avantageux de procéder, dans le cas du maquillage des cils, à l'application d'au moins une desdites compositions avec une brosse de maquillage telle que décrite dans les brevets FR 2 701 198, FR 2 605 505, EP 792 603 et EP 663 161.
Le kit de maquillage selon l'invention peut, selon un mode de réalisation particulier, comprendre au moins deux conditionnements distincts, l'un comprenant la composition cosmétique défmie plus haut et l'autre comprenant la composition aqueuse également définie plus haut.
En réalité, notamment dans le cas du maquillage de fibres kératiniques, celui-ci étant effectué grâce à un geste multiple de l'utilisatrice, à savoir au moins en deux étapes, la première consistant en l'application de la composition "basecoat" et la deuxième consistant en l'application de la composition "topcoat" en tout ou partie sur ladite composition cosmétique, un kit de maquillage conditionné dans un seul et même conditionnement est particulièrement adapté. Cette alternative constitue un mode de réalisation préféré de l'invention.
Lorsque le kit est sous forme d'un seul et même conditionnement, il peut se présenter comme un récipient délimitant au moins un compartiment ou réservoir qui comprend la composition "basecoat", ledit compartiment étant fermé par un élément de fermeture et au moins un compartiment ou réservoir qui comprend la composition "topcoat", étant également fermé par un élément de fermeture.
Toujours lorsque le kit est sous forme d'un seul et même conditionnement, celui-ci est de préférence associé à au moins un moyen d'application ou applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.
L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.
Le récipient, comportant avantageusement deux compartiments ou réservoirs, peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou

alliage).

Le récipient est de préférence équipé d'un essoreur disposé au voisinage d'au moins une ouverture du récipient. Un tel essoreur permet d'essuyer l'applicateur et éventuellement, la tige dont il peut être solidaire. Un tel essoreur est décrit par exemple dans le brevet FR 2 792 618.

Selon un mode de réalisation particulièrement préféré, le kit de maquillage comprend deux réservoirs comprenant chacun l'une des compositions "basecoat" et "topcoat", chacun des réservoirs étant muni d'une brosse de maquillage, notamment de type brosse mascara telle que décrite ci-dessus.

Les exemples qui suivent sont présentés à titre illustratif et non limitatif de l'invention. Sauf indication contraire, les quantités sont données en gramme.

Les tests suivants ont été utilisés pour l'évaluation du gonflement.

<u>Eprouvette et maquillage</u>

On a réalisé des éprouvettes de faux-cils avec des cheveux caucasiens raides noirs de 19 mm de longueur de frange. On a monté lesdites franges entre deux plaques de 30 mm sur 30 mm.

<u>Descriptif du test</u>

On a maquillé les cheveux en 2 étapes :

- Etape 1 : 30 passages de brosse à mascara avec la composition cosmétique à phase continue huileuse dite "basecoat",
- Etape 2 : 20 passages de brosse à mascara avec la composition aqueuse dite "topcoat".

On a évalué le volume de la base seule avant gonflement ($V_b$) après l'étape 1 par analyse photographique et le volume après gonflement après application de la base et du topcoat ($V_{b+t}$) après l'étape 2 également par analyse photographique.

**<u>Exemple 1</u> : Mascara sans cire**

Les résultats relatifs au gonflement ainsi que les teneurs des constituants de compositions de type composition cosmétique à phase continue huileuse conformes à l'invention sont rassemblés dans le tableau 1 ci-après.

La composition aqueuse, dite "topcoat" est un gel aqueux d'hydroxyéthylcellulose à 3,3 % en poids.

<u>Tableau 1</u>

| N° d'ESSAI Base | 0 (comparatif) | 1 (comparatif) | 2 (invention) | 3 (comparatif) | 4 (invention) |
|---|---|---|---|---|---|
| Copolymère Vinyl Acétale/ Allyl stearate | 30 | 30 | 30 | 30 | 30 |
| Colorant | 5,7 | 5,7 | 5,7 | 5,7 | 5,7 |
| Bentone | 3,7 | 3,7 | 3,7 | 3,7 | 3,7 |
| Propylène carbonate | 1,2 | 1,2 | 1,2 | 1,2 | 1,2 |
| Isododecane | Qsp 100 | Qsp 100 | Qsp 100 | Qsp 100 | Qsp 100 |
| Polysorbate 80 (Tween 80 V® d'Uniquema) | 0 | 0 | 2 | 0 | 2 |
| Copolymère Acrylamide / AMPS Na (Simulgel 600® de SEPPIC) | 0 | 10 | 10 | 0 | 0 |
| Polyacrylate de sodium (COSMEDIA SP de Cognis) | 0 | 0 | 0 | 4 | 4 |
| Taux de Gonflement $V_{b+t}/V_b$ | 1,2 | 2,0 | 5,3 | 3,3 | 3,8 |

Ces résultats illustrent clairement le gonflement procuré par l'application successive d'une composition cosmétique à phase continue huileuse conforme à l'invention, suivie de l'application d'une composition aqueuse sur les cils.

On constate un gonflement encore amélioré lorsque la composition cosmétique à phase continue huileuse

contient de plus un agent tensioactif.

**Exemple 2 : Kit**

a) Composition à phase continue huileuse (basecoat)

| | % en poids |
|---|---|
| - Cire d'abeille | 8,0 |
| - Copolymère acétate de vinyle/stéarate d'allyle (65/35) | 20,0 |
| - Tetrapolymère acide méthacrylique/méthacrylate de méthyle/ | |
| - Acrylate de butyle/methacrylate de cétyleicosinyle en dispersion aqueuse à 48 % en poids de MA, 6 % propylène glycol (ALLIANZ OPT de Rohm and Haas) | 10,0 |
| - Polybutène (PM 2060) | 1,0 |
| - Carbonate de propylène | 1,2 |
| - Polysorbate 80 (Tween 80 V® d'Uniquema) | 2,0 |
| - Copolymère Acrylamide/AMPS Na dans isohexadécane avec polysorbate 80 (Simulgel 600® de SEPPIC) | 10,0 |
| - Hectorite modifiée distérayl diméthyl ammonium | 3,7 |
| - Poudre de polytetrafluoroethylène (Microslip 519L de Micropowders) | 5,0 |
| - Colorant | 5,7 |
| - Conservateurs | qs |
| - Isododécane | qsp 100 |

b) Composition aqueuse (topcoat)

| | % en poids |
|---|---|
| - Polyuréthane aliphatique en dispersion aqueuse à 38% en matière active | |
| - (Avalure UR 450® de Novéon) | 46,0 |
| - Hydroxyéthylcellulose | 1,5 |
| - Conservateur | qs |
| - Eau | qsp 100 |

On applique sur les cils une première couche de la composition à phase continue huileuse puis une seconde couche de la composition aqueuse. On observe un gonflement du dépôt final sur les cils et un effet volumateur sur les cils.

## Revendications

1. Kit de maquillage et/ou de soin de la peau, des lèvres et des fibres kératiniques comprenant au moins :

    - une composition cosmétique à phase continue huileuse comprenant au moins un polyélectrolyte et au moins un agent tensioactif de HLB supérieur ou égal à 6 à 25 °C, et
    - une composition aqueuse.

2. Kit selon la revendication 1, **caractérisé en ce que** le polyélectrolyte est choisi parmi un copolymère acrylamide/acide 2-méthyl 2-[(1-oxo-2-propényl)amino]1-propanesulfonique, un glycolate d'amidon réticulé sous forme de poudre, un polyacrylate, un copolymère greffé à base d'amidon, les dérivés ionisables de polysaccharides, les acides polyacryliques, les copolymères acide polyacryliques acrylates d'alkyle, l'AMPS (Acide polyacrylamidométhyl propane sulfonique neutralisé partiellement à l'ammoniaque et hautement réticulé), les copolymères AMPS/méthacrylates d'alkyle polyoxyéthylénés (réticulés ou non), et un mélange de ces derniers et de préférence parmi un copolymère acrylamide/acide 2-méthyl 2-[(1-oxo-2-propényl)amino]1-propanesulfonique, un polyacrylate, et leurs copolymères.

3. Kit selon la revendication 1 ou 2, **caractérisé en ce que** le polyélectrolyte est choisi parmi un copolymère acrylamide/acide 2-méthyl 2-[(1-oxo 2-propényl)amino]1-propanesulfonique, un polyacrylate et leurs copolymères.

**4.** Kit selon l'une quelconque des revendications 1 à 3, **caractérisé en ce que** le polyélectrolyte est présent dans une teneur en poids supérieure ou égale à 0,05 %, et de façon préférée supérieure ou égale à 0,1 % et notamment supérieure ou égale à 0,5 % en poids, par rapport au poids total de la composition cosmétique à phase continue huileuse.

**5.** Kit selon l'une quelconque des revendications 1 à 4, **caractérisé en ce que** le polyélectrolyte est présent dans la composition cosmétique à phase continue huileuse de préférence dans une teneur allant de 0,05 à 15 % en poids, de façon encore plus préférée de 0,1 à 10 % en poids, et mieux encore de 0,5 à 5 % en poids par rapport au poids total de la composition à phase continue huileuse.

**6.** Kit selon l'une quelconque des revendications 1 à 5, **caractérisé en ce que** la composition cosmétique à phase continue huileuse comprend une quantité d'huile totale variant de 10 à 90 %, de préférence de 15 à 80 %, et mieux de 20 à 60 % en poids, par rapport au poids total de la composition à phase continue huileuse.

**7.** Kit selon l'une quelconque des revendications 1 à 6, **caractérisé en ce que** la ou les huiles peu(ven)t être choisie (s) parmi les huiles volatiles et/ou les huiles non volatiles, et leurs mélanges.

**8.** Kit selon la revendication 7, **caractérisé en ce que** les huiles volatiles peuvent être des huiles hydrocarbonées, des huiles siliconées, des huiles fluorées ou leurs mélanges.

**9.** Kit selon l'une quelconque des revendications précédentes **caractérisé en ce que** l'agent tensioactif est présent en une teneur allant de 0,1 à 30 % en poids, mieux de 0,5 à 15 % en poids et mieux de 1,5 à 10 % en poids par rapport au poids total de la composition cosmétique à phase continue huileuse.

**10.** Kit selon l'une quelconque des revendications précédentes, **caractérisé en ce que** la composition cosmétique à phase continue huileuse comprend en outre un agent structurant pouvant être choisi parmi les cires, les polymères semi-cristallins et les gélifiants lipophiles.

**11.** Kit selon l'une quelconque des revendications précédentes, **caractérisé en ce que** la composition cosmétique à phase continue huileuse comprend en outre un polymère filmogène.

**12.** Kit selon la revendication précédente, **caractérisé en ce que** le polymère filmogène est un polymère liposoluble, un polymère hydrosoluble, un polymère d'origine naturelle, un polymère sous forme dispersée en phase aqueuse ou en phase huileuse, et de préférence un polymère en dispersion aqueuse.

**13.** Kit selon l'une quelconque des revendications précédentes, **caractérisé en ce que** la composition cosmétique à phase continue huileuse comprend de l'eau et/ou au moins un solvant hydrosoluble en une teneur inférieure à 30 % en poids, de préférence à 25 %, ou encore à 20 %, notamment à 15 % en poids, par rapport au poids total de la composition cosmétique à phase continue huileuse.

**14.** Kit selon l'une quelconque des revendications précédentes, **caractérisé en ce que** la composition aqueuse contient au moins un polymère filmogène.

**15.** Kit selon la revendication précédente, **caractérisé en ce que** le polymère filmogène est un polymère en dispersion aqueuse.

**16.** Kit selon l'une quelconque des revendications précédentes, **caractérisé en ce que** la composition aqueuse contient un agent gélifiant tel qu'un dérivé cellulosique notamment de l'hydroxyéthylcellulose.

**17.** Kit selon l'une quelconque des revendications précédentes, **caractérisé en ce que** la composition aqueuse est transparente ou translucide.

**18.** Kit selon l'une quelconque des revendications précédentes, **caractérisé en ce que** la composition cosmétique et la composition aqueuse comprennent indépendamment l'une de l'autre en outre une matière colorante, des charges et/ou des fibres.

**19.** Kit selon l'une quelconque des revendications précédentes, **caractérisé en ce que** le kit est sous la forme de rouges à lèvres ou de produits de teint, notamment du type fond de teint, ou encore de mascara.

**20.** Kit selon l'une quelconque des revendications précédentes, **caractérisé en ce qu'**il comprend un ou plusieurs moyens d'application des compositions cosmétiques à phase continue huileuse et aqueuse sur la surface à maquiller.

**21.** Kit selon l'une quelconque des revendications précédentes, **caractérisé en ce qu'**il comprend au moins deux conditionnements distincts, l'un comprenant la composition cosmétique à phase continue huileuse et l'autre comprenant la composition aqueuse.

**22.** Kit selon l'une quelconque des revendications précédentes, **caractérisé en ce qu'**il se présente dans un seul et même conditionnement, notamment sous forme d'un récipient délimitant au moins un compartiment qui comprend la composition cosmétique à phase continue huileuse et au moins un compartiment qui comprend la composition aqueuse, lesdits compartiments étant éventuellement fermés par un élément de fermeture.

**23.** Kit selon l'une quelconque des revendications précédentes, **caractérisé en ce qu'**il s'agit d'un mascara comprenant au moins un moyen d'application, notamment une brosse ou un peigne.

**24.** Procédé cosmétique de maquillage et/ou de soin non thérapeutique de la peau, des lèvres et/ou des fibres kératiniques, **caractérisé en ce qu'**il comprend au moins une étape d'application sur la peau, les lèvres et/ou les fibres kératiniques, notamment les cils, d'une composition cosmétique à phase continue huileuse telle que définie selon l'une quelconque des revendications 1 à 13 et 18 et au moins une étape d'application d'une composition aqueuse telle que définie selon l'une quelconque des revendications 14 à 18.

**25.** Procédé selon la revendication précédente, **caractérisé en ce que** la composition cosmétique à phase continue huileuse est appliquée préalablement à la composition aqueuse.

**26.** Utilisation d'au moins un polyélectrolyte et au moins un agent tensioactif de HLB supérieur ou égal à 6 à 25°C pour la préparation d'un kit de maquillage de la peau, des lèvres et/ou des fibres kératiniques comprenant au moins une composition cosmétique à phase continue huileuse et au moins une composition aqueuse, à titre d'additifs pour ladite composition cosmétique à phase continue huileuse pour obtenir un effet volumateur lorsque lesdites compositions sont mises en contact, par exemple au cours du maquillage.

## Claims

**1.** Kit for making up and/or caring for the skin, the lips and/or keratin fibres, comprising at least:

- one cosmetic composition with an oily continuous phase comprising at least one polyelectrolyte and at least one surfactant having an HLB of greater than or equal to 6 at 25°C, and
- one aqueous composition.

**2.** Kit according to claim 1, **characterized in that** the polyelectrolyte is chosen from a crosslinked starch glycolate in powder form, a polyacrylate, a starch-based grafted copolymer, ionizable polysaccharide derivatives, polyacrylic acids, polyacrylic acid/alkyl acrylate copolymers, AMPS (polyacrylamidomethylpropanesulfonic acid partially neutralized with ammonia and highly crosslinked), AMPS/alkyl methacrylate polyoxyethylenated copolymers (crosslinked or non-crosslinked), and a mixture thereof and preferably from an acrylamide/2-methyl-2-[(1-oxo-2-propenyl)amino]-1-propanesulfonic acid copolymer, a polyacrylate, and their copolymers.

**3.** Kit according to claim 1 or 2, **characterized in that** the polyelectrolyte is chosen from an acrylamide/2-methyl-2-[(1-oxo-2-propenyl)amino]-1-propanesulfonic acid copolymer and a polyacrylate, and copolymers thereof.

**4.** Kit according to anyone of claims 1 to 3, **characterized in that** the polyelectrolyte is present in a weight content of greater than or equal to 0.05%, and preferably of greater than or equal to 0.1% and in particular greater than or equal to 0.5% by weight relative to the total weight of the cosmetic composition with an oily continuous phase.

**5.** Kit according to anyone of claims 1 to 4, **characterized in that** the polyelectrolyte is present in the cosmetic composition with an oily continuous phase in a content ranging from 0.05% to 15% by weight, preferably from 0.1 to 10% by weight and more preferably from 0.5 to 5% by weight relative to the total weight of the composition with an oily continuous phase.

6. Kit according to anyone of claims 1 to 5, **characterized in that** the cosmetic composition with an oily continuous phase comprises a total amount of oil ranging from 10% to 90%, preferably from 15 to 80% and more preferably for 20 to 60% by weight relative to the total weight of the composition with an oily continuous phase.

7. Kit according to anyone of claims 1 to 6, **characterized in that** the oil(s) may be chosen from volatile oils and/or non-volatile oils, and mixtures thereof.

8. Kit according to claim 7, **characterized in that** the volatile oils may be hydrocarbon-based oils, silicone oils or fluoro oils, or mixtures thereof.

9. Kit according to anyone of the preceding claims, **characterized in that** the surfactant is present in a content ranging from 0.1% to 30%, preferably from 0.5 to 15% and more preferably from 1.5 to 10% by weight relative to the total weight of the cosmetic composition with an oily continuous phase.

10. Kit according to anyone of the preceding claims, **characterized in that** the cosmetic composition with an oily continuous phase further comprises a structuring agent that may be chosen from waxes, semi-crystalline polymers and lipophilic gelling agents.

11. Kit according to anyone of the preceding claims, **characterized in that** the cosmetic composition with an oily continuous phase further comprises a film-forming polymer.

12. Kit according to the preceding claim, **characterized in that** the film-forming polymer is a liposoluble polymer, a water-soluble polymer, a polymer of natural origin or a polymer in dispersed form in an aqueous phase or in an oily phase.

13. Kit according to anyone of the preceding claims, **characterized in that** the cosmetic composition with an oily continuous phase comprises water and/or at least one water-soluble solvent in a content of less than 30% by weight preferably less than 25% or even less than 20%, in particular less than 15%, relative to the total weight of the cosmetic composition with an oily continuous phase.

14. Kit according to anyone of the preceding claims, **characterized in that** the aqueous composition contains at least one film-forming polymer.

15. Kit according to the preceding claim, **characterized in that** the film-forming polymer is a polymer in aqueous dispersion.

16. Kit according to anyone of the preceding claims, **characterized in that** the aqueous composition contains a gelling agent such as a cellulose derivative in particular hydroxyethylcellulose.

17. Kit according to anyone of the preceding claims, **characterized in that** the aqueous composition is transparent or translucent.

18. Kit according to anyone of the preceding claims, **characterized in that** the cosmetic composition and the aqueous composition further comprise, independently of each other, a dyestuff, fillers and/or fibres.

19. Kit according to anyone of the preceding claims, **characterized in that** the kit is in the form of lipsticks or complexion products or alternatively mascaras.

20. Kit according to anyone of the preceding claims, **characterized** that it comprises one or more means for applying the cosmetic composition with an oily continuous phase and the aqueous composition to the surface to be made up.

21. Kit according to anyone of the preceding claims, **characterized** that it comprises at least two separate packagings, one comprising the cosmetic composition with an oily continuous phase and the other comprising the aqueous composition.

22. Kit according to anyone of the preceding claims, **characterized** that it presents in a single packaging, in particular in the form of a container delimiting at least one compartment that comprises the cosmetic composition with an oily continuous phase and at least one compartment that comprises the aqueous composition, said compartments being

possibly closed by means of a closing member.

**23.** Kit according to anyone of the preceding claims, **characterized in that** it is a mascara comprising at least one application means, in particular a brush or a comb.

**24.** Cosmetic process for making up and/or caring for the skin, the lips and/or keratin fibres, **characterized** that it is comprises at least one step of applying to the skin, the lips and/or keratin fibres in particular the eyelashes, a cosmetic composition with an oily continuous phase as defined according to any one of claims 1 to 13 and 18, and at least one step of applying an aqueous composition as defined according to anyone of claims 14 to 18.

**25.** Process according to the preceding claim, **characterized in that** the cosmetic composition with an oily continuous phase is applied before the aqueous composition.

**26.** Use of at least one polyelectrolyte and at least one surfactant having an HLB of greater or equal to 6 à 25°C for the preparation of a kit for making up the skin, the lips and/or keratin fibres, comprising at least one cosmetic composition with an oily continuous phase and at least one aqueous composition as an additive for the said cosmetic composition with an oily continuous phase for obtaining a volumizing effect when the said compositions are placed in contact, for example during make-up.

**Patentansprüche**

**1.** Einheit zum Schminken und/oder für die Pflege der Haut, der Lippen und der Keratinfasern, die wenigstens umfasst:

- eine kosmetische Zusammensetzung mit kontinuierlicher öliger Phase, die wenigstens einen Polyelektrolyten und wenigstens ein grenzflächenaktives Mittel mit einem HLB größer oder gleich 6 bis 25 °C enthält, und
- eine wässrige Zusammensetzung.

**2.** Einheit nach Anspruch 1, **dadurch gekennzeichnet, dass** der Polyelektrolyt gewählt ist aus Acrylamid/2-Methyl-2-[(1-Oxo-2-Propenyl)Amino]1-Propansulfonsäure-Copolymer, vernetztem Amidon-Glycolat in Pulverform, Polyacrylat, veredeltem Copolymer auf Amidon-Basis, ionisierbaren Derivaten von Polysacchariden, Polyacrylsäuren, Polyacrylsäure-Alkylacrylat-Copolymeren, AMPS-(Polyacrylamidomethyl-Propansulfonsäure, die teilweise mit Ammoniak neutralisiert und stark vernetzt ist), AMPS/Alkylmethacrylat-Polyoxyethlyen-Copolymeren (vernetzt oder nicht) und einem Gemisch dieser Letzteren und vorzugsweise aus einem Acrylamid/2-Methyl-2-[(1-Oxo-2-Propenyl) Aminio]1-Propansulfonsäure-Copolymer, Polyacrylat und ihren Copolymeren.

**3.** Einheit nach Anspruch 1 oder 2, **dadurch gekennzeichnet, dass** der Polyelektrolyt gewählt ist aus Acrylamid/2-Methyl-2-[(1-Oxo-2-Propenyl)Amino]1-Propansulfonsäure-Copolymer, Polyacrylat und ihren Copolymeren.

**4.** Einheit nach einem der Ansprüche 1 bis 3, **dadurch gekennzeichnet, dass** der Polyelektrolyt in einem Gewichtsanteil größer oder gleich 0,05 Gew.-% und vorzugsweise größer oder gleich 0,1 Gew.-% und insbesondere größer oder gleich 0,5 Gew.- % in Bezug auf das Gesamtgewicht der kosmetischen Zusammensetzung in der kontinuierlichen öligen Phase vorhanden ist.

**5.** Einheit nach einem der Ansprüche 1 bis 4, **dadurch gekennzeichnet, dass** der Polyelektrolyt in einer kosmetischen Zusammensetzung in kontinuierlicher öliger Phase vorzugsweise in einem Gehalt im Bereich von 0,05 bis 15 Gew.-%, stärker bevorzugt im Bereich von 0,1 bis 10 Gew.-% und noch besser im Bereich von 0,5 bis 5 Gew.-% in Bezug auf das Gesamtgewicht der Zusammensetzung mit kontinuierlicher öliger Phase vorhanden ist.

**6.** Einheit nach einem der Ansprüche 1 bis 5, **dadurch gekennzeichnet, dass** die kosmetische Zusammensetzung mit kontinuierlicher öliger Phase einen Gesamtölanteil im Bereich von 10 bis 90 Gew.-%, vorzugsweise im Bereich von 15 bis 80 Gew.- % und besser im Bereich von 20 bis 60 Gew.-% in Bezug auf das Gesamtgewicht der Zusammensetzung mit kontinuierlicher öliger Phase enthält.

**7.** Einheit nach einem der Ansprüche 1 bis 6, **dadurch gekennzeichnet, dass** das oder die Öle gewählt sein können unter flüchtigen Ölen und/oder nichtflüchtigen Ölen und ihren Gemischen.

**8.** Einheit nach Anspruch 7, **dadurch gekennzeichnet, dass** die flüchtigen Öle Kohlenwasserstofföle, Silikonöle,

Fluoröle und ihre Gemische sein können.

9. Einheit nach einem der vorhergehenden Ansprüche, **dadurch gekennzeichnet, dass** das grenzflächenaktive Mittel in einem Anteil im Bereich von 0,1 bis 30 Gew.-%, besser im Bereich von 0,5 bis 15 Gew.-% und noch besser im Bereich von 1,5 bis 10 Gew.-% in Bezug auf das Gesamtgewicht der kosmetischen Zusammensetzung in kontinuierlicher öliger Phase vorhanden ist.

10. Einheit nach einem der vorhergehenden Ansprüche, **dadurch gekennzeichnet, dass** die kosmetische Zusammensetzung in kontinuierlicher öliger Phase außerdem ein strukturbildendes Mittel enthält, das gewählt sein kann aus Wachsen, halbkristallinen Polymeren und lipophilen Quellmitteln.

11. Einheit nach einem der vorhergehenden Ansprüche, **dadurch gekennzeichnet, dass** die kosmetische Zusammensetzung mit kontinuierlicher öliger Phase außerdem ein filmbildendes Polymer enthält.

12. Einheit nach dem vorhergehenden Anspruch, **dadurch gekennzeichnet, dass** das filmbildende Polymer ein fettlösliches Polymer, ein wasserlösliches Polymer, ein Polymer mit natürlichem Ursprung, ein Polymer in in wässriger Phase oder in öliger Phase dispergierter Form und vorzugsweise ein Polymer in wässriger Dispersion ist.

13. Einheit nach einem der vorhergehenden Ansprüche, **dadurch gekennzeichnet, dass** die kosmetische Zusammensetzung mit kontinuierlicher öliger Phase Wasser und/oder wenigstens ein wasserlösliches Lösungsmittel in einem Anteil kleiner als 30 Gew.-%, vorzugsweise kleiner als 25 Gew.-%, stärker bevorzugt kleiner als 20 Gew.- % und insbesondere kleiner als 15 Gew.-% in Bezug auf das Gesamtgewicht der kosmetischen Zusammensetzung mit kontinuierlicher öliger Phase enthält.

14. Einheit nach einem der vorhergehenden Ansprüche, **dadurch gekennzeichnet, dass** die wässrige Zusammensetzung wenigstens ein filmbildendes Polymer enthält.

15. Einheit nach dem vorhergehenden Anspruch, **dadurch gekennzeichnet, dass** das filmbildende Polymer ein Polymer in wässriger Dispersion ist.

16. Einheit nach einem der vorhergehenden Ansprüche, **dadurch gekennzeichnet, dass** die wässrige Zusammensetzung ein Quellmittel enthält, das ein Zellulosederivat, insbesondere Hydroxyethyl-Zellulose ist.

17. Einheit nach einem der vorhergehenden Ansprüche, **dadurch gekennzeichnet, dass** die wässrige Zusammensetzung transparent oder klar ist.

18. Einheit nach einem der vorhergehenden Ansprüche, **dadurch gekennzeichnet, dass** die kosmetische Zusammensetzung und die wässrige Zusammensetzung unabhängig voneinander außerdem einen Farbstoff, Ballastmittel und/oder Fasern enthalten.

19. Einheit nach einem der vorhergehenden Ansprüche, **dadurch gekennzeichnet, dass** die Einheit in Form von Lippen-Rouge oder von Teint-Produkten, insbesondere Makeup oder aber Wimperntusche vorliegt.

20. Einheit nach einem der vorhergehenden Ansprüche, **dadurch gekennzeichnet, dass** sie ein oder mehrere Mittel zum Anwenden der kosmetischen Zusammensetzungen mit kontinuierlicher öliger oder wässriger Phase auf die zu schminkende Oberfläche umfasst.

21. Einheit nach einem der vorhergehenden Ansprüche, **dadurch gekennzeichnet, dass** sie wenigstens zwei verschiedene Verpackungen umfasst, wovon eine die kosmetische Zusammensetzung mit kontinuierlicher öliger Phase die andere die wässrige Zusammensetzung enthält.

22. Einheit nach einem der vorhergehenden Ansprüche, **dadurch gekennzeichnet, dass** sie in ein und derselben Verpackung vorliegt, insbesondere in Form eines Aufnahmebehälters, der wenigstens ein Fach, das die kosmetische Zusammensetzung mit kontinuierlicher öliger Phase enthält, und wenigstens ein Fach, das die wässrige Zusammensetzung enthält, begrenzt, wobei die Fächer eventuell durch ein Verschlusselement verschlossen sind.

23. Einheit nach einem der vorhergehenden Ansprüche, **dadurch gekennzeichnet, dass** es sich um eine Wimperntusche handelt, die wenigstens ein Aufbringungsmittel, insbesondere eine Bürste oder einen Kamm, enthält.

**24.** Kosmetisches Verfahren zum Schminken und/oder für die therapeutische Pflege der Haut, der Lippen und/oder der Keratinfasern, **dadurch gekennzeichnet, dass** es wenigstens einen Schritt des Aufbringens einer kosmetischen Zusammensetzung mit kontinuierlicher öliger Phase nach einem der Ansprüche 1 bis 13 und 18 auf die Haut, die Lippen und/oder die Keratinfasern, insbesondere die Wimpern, und wenigstens einen Schritt des Aufbringens einer wässrigen Lösung nach einem der Ansprüche 14 bis 18 umfasst.

**25.** Verfahren nach dem vorhergehenden Anspruch, **dadurch gekennzeichnet, dass** die kosmetische Zusammensetzung mit kontinuierlicher öliger Phase vor der wässrigen Zusammensetzung aufgebracht wird.

**26.** Verwendung wenigstens eines Polyelektrolyten und wenigstens eines grenzflächenaktiven Mittels mit einem HLB größer oder gleich 6 bis 25 °C für die Zubereitung einer Einheit zum Schminken der Haut, der Lippen und/oder der Keratinfasern, die wenigstens eine kosmetische Zusammensetzung mit kontinuierlicher öliger Phase und wenigstens eine wässrige Zusammensetzung als Additiv für die kosmetische Zusammensetzung mit kontinuierlicher öliger Phase enthält, um eine Volumenwirkung zu erhalten, wenn die Zusammensetzungen in Kontakt gebracht werden, beispielsweise während des Schminkens.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6045783 A
- EP 1228747 A
- FR 2785801
- EP 1152022 A
- FR 2774996
- WO 9535089 A
- WO 9936445 A
- WO 9952499 A
- EP 847752 A
- FR 2792190 A
- EP 1396259 A
- US 5156911 A
- WO 0119333 A
- FR 2232303 A
- US 5874069 A
- US 5919441 A
- US 6051216 A
- US 5981680 A
- EP 1411069 A
- WO 04028488 A
- US 5188899 A
- WO 04055081 A
- FR 2701198
- FR 2605505
- EP 792603 A
- EP 663161 A
- US 4887622 A
- FR 2796529
- FR 2761959
- FR 2792618


### Littérature non-brevet citée dans la description


- **GRIFFIN.** *J. Soc. Cosm. Chem.,* 1954, vol. 5, 249-256
- Encyclopedia of Chemical Technology, KIRK-OTHMER. WILEY, 1979, vol. 22, 333-432
- **VAN DE HULST, H.C.** Light Scattering by Small Particles. Wiley, 1957
- **S. NOJIMA.** Melting behavior of poly(ε-caprolactone)-block-polybutadiène copolymers. *Macromolécules,* 1999, vol. 32, 3727-3734
- **B. BOUTEVIN et al.** Study of morphological and mechanical properties of PP/PBT. *Polymer Bulletin,* 1995, vol. 34, 117-123
- **P. RANGARAJAN et al.** Morphology of semi-crystalline block copolymers of ethylene-(ethylene-alt-propylene. *Macromolecules,* 1993, vol. 26, 4640-4645
- **P. RICHTER et al.** Polymer agregates with crystalline cores : the system poly(ethylene)-poly(ethylene-propylene. *Macromolécules,* 1997, vol. 30, 1053-1068
- **I.W. HAMLEY.** Cristallization in block copolymers. *Advances in Polymer Science,* 1999, vol. 148, 113-137
- Silica silylate. CTFA. 1995
- Silica diméthyl silylate. CTFA. 1995